# EUROPEAN PATENT SPECIFICATION

(11) **EP 1 872 755 B1**
(45) Date of publication and mention of the grant of the patent: **17.11.2010**
(21) Application number: 07111414.4
(22) Date of filing: 29.06.2007
(51) Int. Cl.: A61F 9/008, G02B 26/08

(54) **System for steering an optical beam**
System zur Lenkung eines optischen Strahls
Système pour l'orientation d'un faisceau optique

(30) Priority: 30.06.2006 US 817952 P
(43) Date of publication of application: 02.01.2008
(73) Proprietor: Alcon, Inc., 6331 Hünenberg (CH)
(72) Inventor: Horvath, Christopher, Irvine CA 02604 (US); Romoda, Laszlo O., San Clemente CA 92673 (US)
(74) Representative: Moore, Barry

(56) References cited:
- EP-A2- 0 867 151
- DE-C1- 4 106 565
- JP-A- 57 163 201
- US-A- 4 477 159

## Description

The present invention relates to optical beam steering devices. More particularly, the present invention relates to optical beam steering devices in laser systems used in ophthalmic surgical systems. Even more particularly, the present invention relates to a multiple discreet position mirror assembly for directing a surgical laser beam into multiple outlet ports.

### BACKGROUND OF THE INVENTION

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreo-retinal surgery, and anterior segment procedures, such as cataract surgery. More recently, combined anterior and posterior segment procedures have been developed.

The surgical instrumentation used for ophthalmic surgery can be specialized for anterior segment procedures or posterior segment procedures or support both. In any case, the surgical instrumentation often implements a whole host of functionality which may be used in the implementation of a wide variety of surgical procedures.

Laser surgery to the retina is the standard of care in the treatment of numerous ophthalmic diseases. Diseases treated by laser photocoagulation include proliferative diabetic retinopathy, diabetic macular edema, cystoid macular edema, retinal vein occlusion, choroidal neovascularization, central serous chorioretinopathy, retinal tears, and other lesions.

As may be imagined, the complexities of these types of retina surgeries may be quite variegated, and concomitantly, the surgical devices used to conduct these surgeries may need to implement a whole host of functionality associated with these surgeries. Often, a surgical laser system may be operable to implement functionality associated with multiple types of surgeries or other procedures, such that one surgical laser system may be used in multiple types of operations or procedures. In particular, a useful feature to have in such surgical laser systems is multiple laser output ports to support connection of multiple surgical laser probes. Multiple laser output ports, and the multiple surgical laser probes they allow for, permit a surgeon added flexibility and surgical efficiency to switch between different procedures/adaptations without the interruption caused by having to disconnect one probe and connect another. Because it is undesirable, however, from both a cost, complexity and safety perspective, to have either multiple surgical lasers to supply a dedicated laser beam to each port, or to split a single laser beam into multiple dedicated paths for each laser output port, such multi-port surgical laser systems require a reliable switching mechanism to alternatively steer a single laser beam among multiple outputs.

A high level of precision and stability is required to focus a laser beam into a fiber launch (output port). To be able to steer a beam to different output points requires that the precision be repeatable. Prior art beam deflecting mounts traditionally rotate the reflective surface of a mirror to a desired angle and tilt to deliver a laser beam to a desired location. The beam deflecting mount motion can be either active or passive. Active systems use motorized control systems with active feedback to control angle and tilt. Passive systems are simple and use a simple motion device, such as a solenoid with hard stops, to set the desired angle and tilt of a reflective mirror surface. A problem with either of these solutions is that both move the mirror's reflective surface in the plane of the laser beam they are steering and thus must precisely control the location of the reflective surface along multiple degrees of freedom. To achieve this level of precision, these prior art systems typically require precise beam locating schemes using encoders, active feedback loops, and rotational devices with precise stops, resulting in complex and expensive configurations that are constantly correcting the reflecting surface position during operation, and hence are prone to error and/or require frequent adjustment.

US 4477159 discloses an ophthalmic system with a rotatable prism as beam steering element.

JP 57 163201 describes an apparatus for steering a light beam which comprises first and second reflecting surfaces which are integrally formed with one another.

Therefore, a need exists for a multiple discreet position mirror assembly for directing a laser beam along multiple optical paths that can reduce or eliminate the problems of prior art beam steering devices and systems.

### BRIEF SUMMARY OF THE INVENTION

These and other needs are addressed in accordance with the teaching of the invention by a system as claimed in claim 1. Advantageous embodiments are provided in the dependent claims. The scope of the invention is as defined by the claims.

The multiple optical paths caneach then be an optical path leading to an outlet port wherein the light beam is directed into an optical fiber of a handheld probe. Such systems will be familiar to those having skill in the art. The first and second reflective surfaces should move only in a linear manner and have optically negligible to no rotational movement (i.e., the first and second pre-defined angles remain fixed as necessary to prevent unwanted deviation of the light beam).

Other embodiments of the system for directing a light beam along multiple optical paths of this invention can comprise a multi-port surgical console for controlling the surgical laser. For example, embodiments of the present invention can be implemented within any ophthalmic surgical system having multiple output ports/optical paths as may be familiar to those having skill in the art, such as the NGL Laser Surgical System manufactured by Alcon Manufacturing, Ltd. of Irvine, California. The embodiments of this invention can be incorporated within any such surgical machine or system for use in ophthalmic or other surgery. Other uses for a system for directing a light beam along multiple optical paths designed in accordance with the teachings of this invention will be known to those having skill in the art and are contemplated to be within the scope of this invention, which is as defined by the claims.

### BRIEF DESCRIPTION OF THE SEVERAL VIEWS OF THE DRAWINGS

A more complete understanding of the present invention and the advantages thereof may be acquired by referring to the following description, taken in conjunction with the accompanying drawings in which like reference numerals indicate like features and wherein:
FIGUREs 1a-1d are functional block diagrams illustrating the operation of one embodiment of a multiple discreet position mirror assembly of this invention;
FIGUREs 2 and 3 are schematic diagrams illustrating the operation of one embodiment of a system for directing a light beam along multiple optical paths of this invention; and
FIGUREs 4 and 5 are close-up schematic diagrams illustrating the operation and linear movement of an embodiment of the apparatus for directing a light beam along multiple optical paths of this invention in greater detail.

### DETAILED DESCRIPTION OF THE INVENTION

Preferred embodiments of the present invention are illustrated in the FIGUREs, like numerals being used to refer to like and corresponding parts of the various drawings.

The various embodiments of the present invention provide a system and apparatus for alternatively directing a light beam along multiple optical paths. Unlike prior art beam steering systems that use a single mirror, or multiple mirrors with complex and sensitive components, precise locating schemes and encoders, feedback loops, and/or rotational devices with precise stops, the embodiments of the present invention have a linear travel design with multiple, fixed reflective surfaces/mirrors that articulate perpendicular to a light beam path with only one required degree of freedom and no adjustment while in operation. The embodiments of this invention comprise a discrete mirror for each optical path on which it is desired to direct the light beam. These mirrors are positioned at a fixed angle to the incident light beam and translate only in a linear direction so as to maintain the fixed angle to the beam. The embodiments of this invention thus reduce the precision required to maintain correct angle and tilt of the reflective surfaces to one degree of freedom that is at a fixed angle to the light beam.

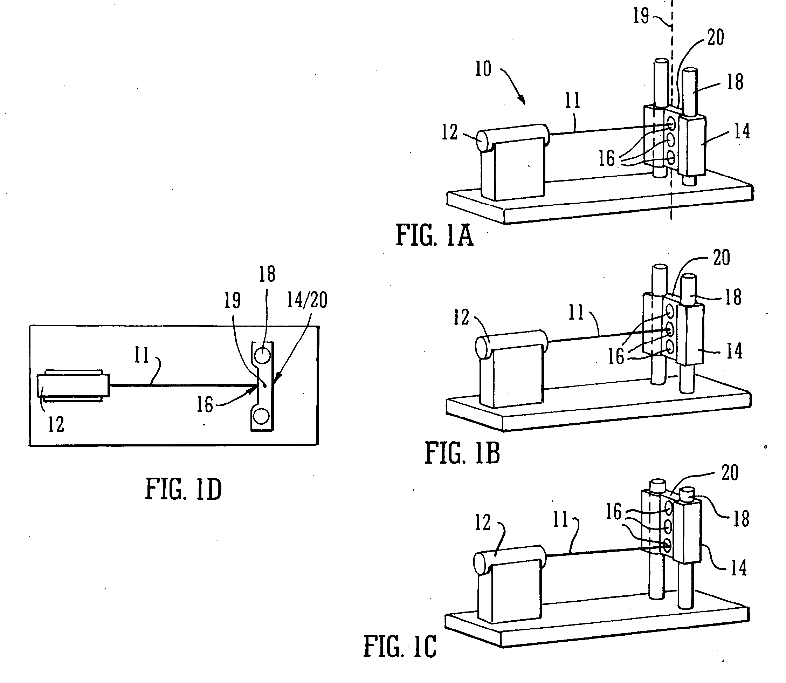

FIGUREs 1a-1d are functional block diagrams illustrating the operation of an embodiment of the multiple discreet position mirror assembly of this invention. Beam steering system 10 comprises a light source 12, which can be a surgical laser, and mirror assembly 14. Mirror assembly 14 comprises a plurality of reflective surfaces 16, which can be precision optical mirrors, and a base assembly 20. Reflective surfaces 16 are each set at a pre-defined and fixed angle to the optical axis of the incident light beam 11 and are operable to direct the light beam 11 along an optical path. Each reflective surface 16 can be fixed at a different pre-defined angle to the incident light beam 11 and can direct the light beam 11 along a different optical path. In a surgical laser system, each optical path could lead to an optical port, wherein the light beam 11 can be optically coupled to a fiber-optic cable within a handheld probe for delivery to a surgical site. This is a preferred use for the embodiments of this invention.

As can be seen in FIGUREs 1a-1d, reflective surfaces 16 are in a fixed relationship to each other and are operable to be moved in a liner manner, in this case perpendicular to the incident light beam 11, along a common axis 19. Mirror assembly 14, to which reflective surfaces 16 can be operably coupled, is operable to linearly reposition the reflective surfaces 16 so that light beam 16 is alternatively incident on one or another of reflective surfaces 16. In this way, the multiple discreet position mirror assembly 16 of this invention can direct incident light beam 16 along a desired path (e.g., to a desired output port). Incident light beam 11 can be a laser beam, such as a surgical laser beam.

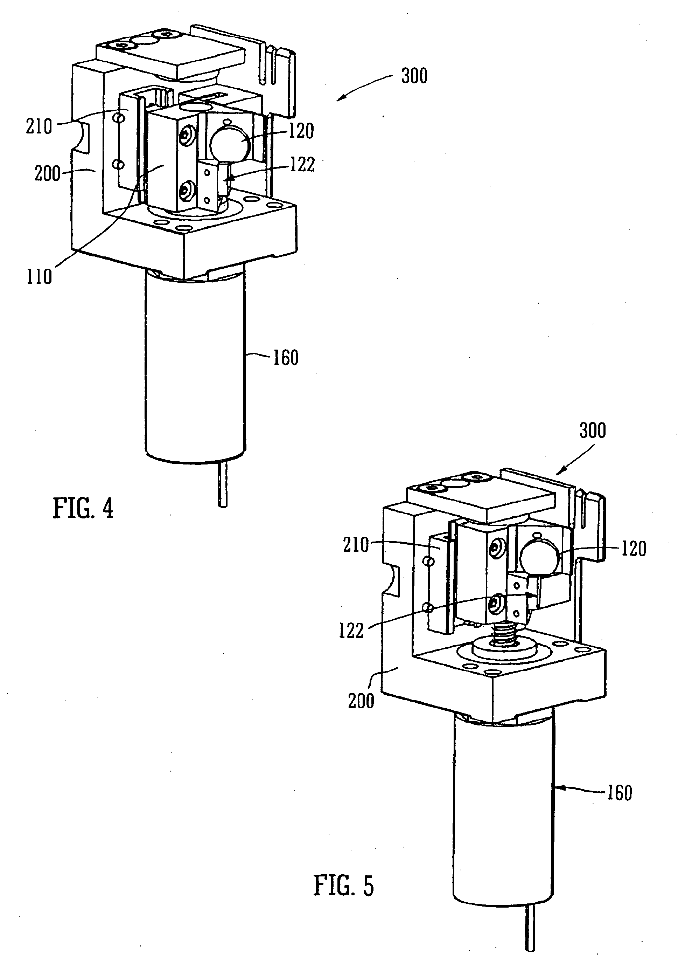

Mirror assembly 14 can be operably attached to mounting 18 via, for example, a ball bearing assembly or other precision sliding mechanism as will be familiar to those having skill in the art. Beam steering system 10 can further include a movement means (as shown in FIGUREs 4 and 5) operably coupled to the mirror assembly 14 to provide motive force to reposition reflective surfaces 16. The movement means can be, for example, a solenoid, or other mechanism for imparting linear motion as will be known to those having skill in the art. The reflective surfaces 16 can be operably coupled to the base assembly 20/mirror assembly 14 and can be integral to the base assembly 20/mirror assembly 14. Base assembly 20 can be integral to or operably attached to mirror assembly 14. Although shown in FIGUREs 1a-1d with three reflective surfaces 16, mirror assembly 16 can comprise more or less reflective surfaces 16 in fixed relationship to one another and operable to direct the light beam 11 along multiple optical paths. In particular, mirror assembly 16, in one embodiment, can have dual reflective surfaces 16 for directing light beam 11 along two optical paths.

The present invention comprises a surgical laser system having multiple output ports, wherein a laser beam (e.g., light beam 11) is directed into a desired port by a beam steering system 10 of FIGUREs 1a-1d. The light beam 11 is optically coupled to a laser probe, such as an endolaser probe, at an output port and can be delivered by the laser probe to a surgical site. In such a system, a port can be selected (mirror assembly 14 positioned) either by a mechanical switching mechanism or a button or other control mechanism on the surgical laser system (e.g., a probe connected to a port can cause a port to be selected and cause mirror assembly 14 to position itself such that incident light beam 11 will be directed to the selected port by the respective reflective surface 16 corresponding to that port's optical path).

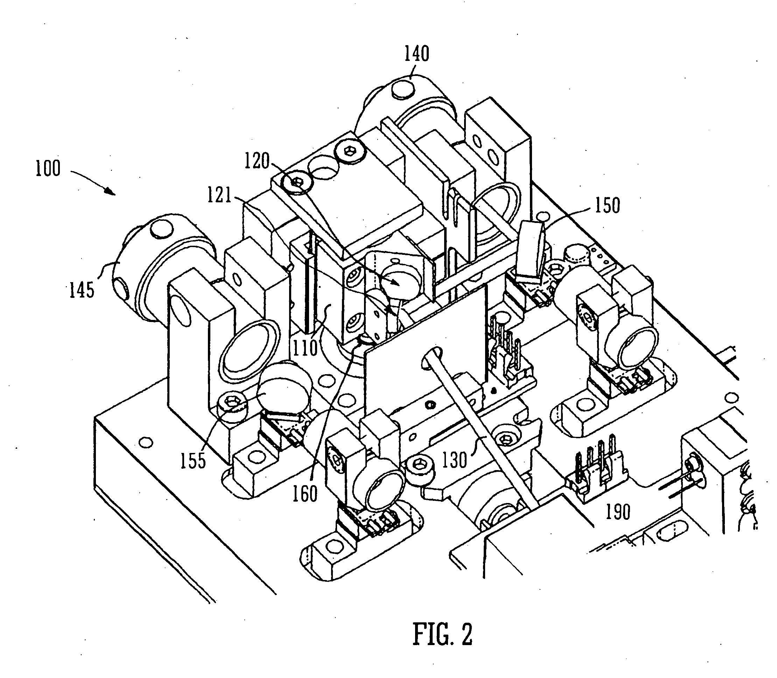

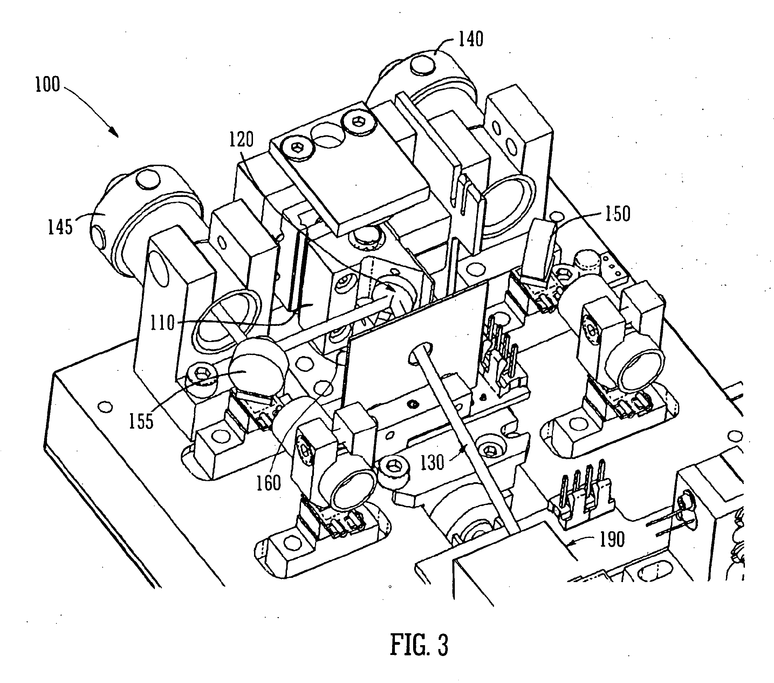

FIGUREs 2 and 3 are schematic drawings illustrating the operation of one embodiment of the system for steering an optical beam along multiple paths of the present invention having dual reflective surfaces and two output ports. Steering system 100 includes mirror/base assembly 110, reflective surfaces 120 and 122 and movement means 160. As shown in FIGUREs 2 and 3, steering system 100 is implemented within an ophthalmic surgical laser system and directs a laser beam 130 from laser 190 along either of two optical paths to either output port 140 or output port 145, depending on the position of mirror assembly 110. As described with reference to mirror assembly 14 of FIGUREs 1a-1d, mirror assembly 110 is operable to move in a linear direction (one degree of freedom) along a common axis to reposition reflective surfaces 120/122 so that one or the other receives incident laser beam 130 and directs the laser beam 130 to either output port 140 or 145.

Although shown in FIGUREs 2 and 3 as being perpendicular to incident laser beam 130, the linear motion of mirror assembly 110 and reflective surfaces 120/122 can be at any angle to the optical axis of incident laser beam 130 as may be needed in a particular application or configuration of system 100 or the laser system in which steering system 100 is implemented. Further, although in FIGUREs 2 and 3 light beam 130 is shown directed onto either steering mirror 150 or 155 prior to arriving at output ports 140/145, reflective surface 120/122 can instead direct laser beam 130 to output port 140/145 without an intervening steering mirror 150/155, or with multiple intervening optical components, depending on the configuration of the optical paths as will be known to those having skill in the art of directing light beams along an optical path Mirror assembly 110/reflective surfaces 120/122 can be moved in a linear direction by movement means 160, which can be a solenoid or any other mechanism capable of imparting linear motion as will be known to those having skill in the art.

Reflective surfaces 120/122 are discrete surfaces that are each set at a pre-defined and fixed angle, which can be different from one another, to the optical axis of incident laser beam 130 and can also be in a fixed relationship to one another. Reflective surfaces 120/122 translate only in a linear manner and at a fixed angle to the laser beam 130. Reflective surfaces 120/122 preferably have optically negligible or no rotational movement (i.e., the angles of reflective surfaces 120/122 to laser beam 130 remain fixed as necessary to prevent unwanted deviation of the laser beam 130).

Laser beam 130, in a typical implementation, is about 2 mm in diameter. Reflective surfaces 120/122 can be about 7 mm in diameter, so as to provide some margin for error in the positioning of reflective surfaces 120/122. Because reflective surfaces 120/122 are at a fixed angle to the optical axis of laser beam 130 and because they move only in a linear manner such that they maintain the ir respective fixed angle to the incident laser beam 130, the diameter difference between the laser beam 130 and reflective surfaces 120/122 are able to provide a margin for positional error of reflective surfaces 120/122 such that laser beam 130 will be directed to a desired location even if reflective surfaces 120/122 are slightly off an intended position. In this way, inaccuracy introduced by the wearing of mirror assembly 110 stops, or any other slight linear error, will not adversely affect steering of laser beam 130. Mirror assembly 110 can thus comprise soft stops at either end of its range of motion, to prevent vibration and mechanical stress from damaging sensitive optical components, while still maintaining required accuracy.

FIGUREs 4 and 5 are close-up schematic diagrams illustrating the operation of an embodiment of the apparatus for directing a light beam along multiple optical paths of the present invention in greater detail. Steering apparatus 300 includes mirror assembly 110, reflective surfaces 120/122, movement means 160 and frame 200. Frame 200 is operable to support and operably couple mirror assembly 110 and movement means 160. Frame 200 includes slide 210 for guiding mirror assembly 110's linear movement. Slide 210 can comprise a precision ball bearing assembly or any precision sliding mechanism as will be known to those having skill in the art and operably to precisely guide mirror assembly 110 in a linear manner as it moves. Movement means 160 can also be operably coupled to mirror assembly 110 by means of precision ball bearings or other precision sliding mechanism as will be known to those having skill in the art and operable to provide smooth and precise movement of mirror assembly 110.

The present invention has been described by reference to certain preferred embodiments; however, it should be understood that it may be embodied in other specific forms or variations thereof without departing from its spirit or essential characteristics. The embodiments described above are therefore considered to be illustrative in all respects and not restrictive, the scope of the invention being indicated by the appended claims. As may be used herein, the terms "substantially" and "approximately" provide an industry-accepted tolerance for their corresponding term and/or relativity between items. Such an industry-accepted tolerance ranges from less than one percent to fifty percent and corresponds to, but is not limited to, component values, integrated circuit process variations, temperature variations, rise and fall times, and/or thermal noise. Such relativity between items ranges from a difference of a few percent to magnitude differences. As may also be used herein, the term(s) "coupled to" and/or "coupling" include direct coupling between items and/or indirect coupling between items via an intervening item (e.g., an item includes, but is not limited to, a component, an element, a circuit, and/or a module) where, for indirect coupling, the intervening item does not modify the information of a signal but may adjust its current level, voltage level, and/or power level. As may further be used herein, inferred coupling (i.e., where one element is coupled to another element by inference) includes direct and indirect coupling between two items in the same manner as "coupled to". As may even further be used herein, the term "operable to" indicates that an item includes one or more of power connections, input(s), output(s), etc., to perform one or more its corresponding functions and may further include inferred coupling to one or more other items. As may still further be used herein, the term "associated with", includes direct and/or indirect coupling of separate items and/or one item being embedded within another item. As may be used herein, the term "compares favorably", indicates that a comparison between two or more items, signals, etc., provides a desired relationship. For example, when the desired relationship is that signal 1 has a greater magnitude than signal 2, a favorable comparison may be achieved when the magnitude of signal 1 is greater than that of signal 2 or when the magnitude of signal 2 is less than that of signal 1.

While the present invention has been described with reference to the general area of laser ophthalmic surgery, the teachings contained herein can apply equally to any surgical system where it is desirous to control a laser subsystem.

## Claims

1. A system for steering a light beam to multiple output ports (140, 145) for connecting multiple surgical laser probes, the laser probes being endolaser probes operable to deliver the light beam to a surgical site, comprising:
a light source comprising an ophthalmic surgical laser, operable to provide the light beam; and
an apparatus (10, 100) for steering a light beam, **characterized in that** it comprises :
a base assembly (20, 110);
a first reflective surface (16, 120) set at a first fixed angle to the optical axis of an incident light beam (11, 130) and operable to direct the light beam (11, 130) along a first optical path; and
a second reflective surface (16,122) set at a second fixed angle, different from the first angle, to the optical axis of the incident light beam and operable to direct the light beam along a second optical path,
wherein the first optical path leads to the first output port (140) and the second optical path leads to the second output port (145) and the first and second output ports are operable to optically couple the light beam to a laser probe,
wherein the first reflective surface and the second reflective surface are operable to move only in a linear manner along a common axis (19)such that the correct angle and tilt of the reflective surfaces to the incident light beam is maintained to one degree of freedom, and wherein the base assembly (20, 110) is operable to linearly reposition the first (16, 120) and second (16, 122) reflective surfaces along the common axis so that the incident light beam is incident on one or the other reflective surfaces, the second reflective surface (16, 122) being discrete from the first reflective surface (16, 120), to effect a selection of one or other of the first optical path or the second optical path as required, with no adjustment while in operation.

2. The system of claim 1, wherein the first and second angles are pre-defined and fixed relative to one another.

3. The system of claim 1, wherein the second optical path is different from the first optical path.

4. The system of claim 1, wherein the first (16, 120) and second (16, 122) reflective surfaces are arranged in a fixed relationship to one another.

5. The system of claim 1, wherein the base assembly comprises a movement means (160) operable to linearly reposition the first and second reflective surfaces.

6. The system of claim 1, wherein the movement means is a solenoid.

7. The system of claim 1, wherein the first and second reflective surfaces are precision mirrors.

8. The system of claim 1, wherein the light beam is a surgical laser beam.

9. The system of claim 1, wherein the surgical site comprises a retina.

## Patentansprüche

1. System zum Lenken eines Lichtstrahls zu mehreren Ausgangsanschlüssen (140, 145), um mehrere chirurgische Lasersonden anzuschließen, wobei es sich bei den Lasersonden um Endo-Lasersonden handelt, die den Lichtstrahl an eine chirurgische Stelle liefern können, mit:
einer Lichtquelle, die einen ophthalmischen chirurgischen Laser aufweist, der den Lichtstrahl bereitstellen kann, und
eine Vorrichtung (10, 100) zum Lenken eines Lichtstrahls,
**dadurch gekennzeichnet, dass** sie aufweist:
eine Basisbaugruppe (20, 110);
eine erste reflektierende Oberfläche (16, 120), die in einem ersten festen Winkel zur optischen Achse eines einfallenden Lichtstrahls (11, 130) eingerichtet ist und den Lichtstrahl (11, 130) entlang einem ersten optischen Weg richten kann; und
eine zweite reflektierende Oberfläche (16, 122), die in einem zweiten festen Winkel, der vom ersten Winkel verschieden ist, zur optischen Achse des einfallenden Lichtstrahls eingerichtet ist, und den Lichtstrahl entlang einem zweiten optischen Weg richten kann,
wobei der erste optische Weg zu einem ersten Ausgangsanschluss (140) und der zweite optische Weg zu einem zweiten Ausgangsanschluss (145) führt, und der erste und
zweite Ausgangsanschluss den Lichtstrahl mit der Lasersonde optisch koppeln können,
wobei sich die erste und die zweite reflektierende Oberfläche nur auf eine lineare Weise entlang einer gemeinsamen Achse (19) so bewegen können, dass der korrekte Winkel und die Neigung der reflektierenden Oberflächen relativ zum einfallenden Lichtstrahl auf einem Freiheitsgrad konstant gehalten werden, und wobei die Basisbaugruppe (20, 110) die erste (16, 120) und die zweite (16, 122) reflektierende Oberfläche entlang der gemeinsamen Achse linear repositionieren kann, so dass der einfallende Lichtstrahl auf die eine oder die andere reflektierende Oberfläche fällt, wobei die zweite reflektierende Oberfläche (16, 122) von der ersten reflektieren Oberfläche (16, 120) verschieden ist,
um den einen oder den anderen des ersten oder zweiten optischen Weges wie erforderlich zu wählen, wobei während des Betriebs keine Verstellung stattfindet.

2. System nach Anspruch 1, bei dem der erste und zweite Winkel vordefiniert und relativ zueinander fest sind.

3. System nach Anspruch 1, bei dem der zweite optische Weg vom ersten optischen Weg verschieden ist.

4. System nach Anspruch 1, bei dem die erste (16, 120) und die zweite (16, 122) reflektierende Oberfläche in einer festen Beziehung zueinander angeordnet sind.

5. System nach Anspruch 1, bei dem die Basisbaugruppe ein Bewegungsmittel (160) aufweist, das die erste und zweite reflektierende Oberfläche linear repositionieren kann.

6. System nach Anspruch 1, bei dem das Bewegungsmittel ein Solenoid ist.

7. System nach Anspruch 1, bei dem die erste und zweite reflektierende Oberfläche Präzisionsspiegel sind.

8. System nach Anspruch 1, bei dem der Lichtstrahl ein chirurgischer Laserstrahl ist.

9. System nach Anspruch 1, bei dem die chirurgische Stelle eine Retina (Netzhaut) ist.

## Revendications

1. Système pour orienter un faisceau lumineux vers de multiples orifices de sortie (140, 145) afin de relier de multiples sondes laser chirurgicales, les sondes laser étant des sondes laser endoscopiques opérationnelles pour délivrer le faisceau lumineux sur un site chirurgical, comprenant :
une source de lumière comprenant un laser chirurgical ophtalmique, opérationnelle pour fournir le faisceau lumineux ; et
un appareil (10, 100) pour orienter un faisceau lumineux, **caractérisé en ce qu'**il comprend :
un ensemble formant base (20, 110) ;
une première surface réflectrice (16, 120) disposée selon un premier angle fixe par rapport à l'axe optique d'un faisceau lumineux incident (11, 130) et opérationnelle pour diriger le faisceau lumineux (11, 130) le long d'un premier trajet optique ; et
une seconde surface réflectrice (16, 122) disposée selon un second angle fixe, différent du premier angle, par rapport à l'axe optique du faisceau lumineux incident et opérationnelle pour diriger le faisceau lumineux le long d'un second trajet optique,
dans lequel le premier trajet optique mène au premier orifice de sortie (140) et le second trajet optique mène au second orifice de sortie (145), et les premier et second orifices de sortie sont opérationnels pour coupler de manière optique le faisceau lumineux à une sonde laser,
dans lequel la première surface réflectrice et la seconde surface réflectrice sont opérationnelles pour se déplacer uniquement de manière linéaire le long d'un axe commun (19) de manière à ce que le bon angle et l'inclinaison des surfaces réflectrices par rapport au faisceau lumineux incident soient maintenus à un degré de liberté, et dans lequel l'ensemble formant base (20, 110) est opérationnel pour repositionner de manière linéaire les première (16, 120) et seconde (16, 122) surfaces réflectrices le long de l'axe commun de manière à ce que le faisceau lumineux incident soit incident sur l'une ou l'autre des surfaces réflectrices, la seconde surface réflectrice (16, 122) étant discrète à partir de la première surface réflectrice (16, 120) pour effectuer une sélection de l'un ou de l'autre des premier et second trajets optiques si nécessaire, sans ajustement tout en étant en fonctionnement.

2. Système selon la revendication 1, dans lequel les premier et second angles sont prédéfinis et fixes l'un par rapport à l'autre.

3. Système selon la revendication 1, dans lequel le second trajet optique est différent du premier trajet optique.

4. Système selon la revendication 1, dans lequel les première (16, 120) et seconde (16, 122) surfaces réflectrices sont agencées de manière fixe l'une par rapport à l'autre.

5. Système selon la revendication 1, dans lequel l'ensemble formant base comprend des moyens de déplacement (160) opérationnels pour repositionner de manière linéaire les première et seconde surfaces réflectrices.

6. Système selon la revendication 1, dans lequel les moyens de déplacement sont un solénoïde.

7. Système selon la revendication 1, dans lequel les première et seconde surfaces réflectrices sont des miroirs de précision.

8. Système selon la revendication 1, dans lequel le faisceau lumineux est un faisceau laser chirurgical.

9. Système selon la revendication 1, dans lequel le site chirurgical comprend une rétine.
